# EUROPEAN PATENT SPECIFICATION

(11) **EP 1 504 781 B1**
(45) Date of publication and mention of the grant of the patent: **15.04.2009**
(21) Application number: 04077213.9
(22) Date of filing: 02.08.2004
(51) Int. Cl.: A61M 15/00

(54) **An inhalator for powder mixtures**
Inhalator für Pulvermischungen
Inhalateur pour des mélanges de poudre

(30) Priority: 07.08.2003 IT MO20030229
(43) Date of publication of application: 09.02.2005
(73) Proprietor: OLIVA, Roberto, 41012 Carpi (Modena) (IT)
(72) Inventor: OLIVA, Roberto, 41012 Carpi (Modena) (IT)
(74) Representative: Brogi, Graziano

(56) References cited:
- EP-A- 0 424 790
- AP-A- 272
- DE-A- 10 106 788
- DE-C- 4 415 462
- US-A- 4 570 630
- US-A- 5 383 850

## Description

The invention relates to an inhalator of a type which contains a determined quantity of a powder mixture which is made available for inhalation in measured doses.

Various types of inhalators are known, which in general comprise a reservoir in which a powder preparation is contained, associated to an inhalation conduit. Means for batching are located between the reservoir and the inhalation conduit, which make available predefined doses of the prepared mixture. The inhalation, by a user, of the predefined dose is done by aspiration from the inhalation conduit. The user places an end of the inhalation conduit by his or her mouth and inhales, up until the whole dose has been ingested.

Known-type inhalators generally exhibit rather complex architectures. The means for dosing, especially, are constituted by numerous components, assembled to create mechanisms which collect a dose of mixture from the reservoir and transfer it to the right position for inhalation through the inhalation conduit. These mechanisms are subject to blockages and obstruction and also increase the total cost of the inhalator.

In known-type devices, at the opening of the inhalation conduit a quite high and prolonged depression is required in order to guarantee total assumption of the mixture dose. The user has to aspirate intensely and for quite a long time in order to be sure of having ingested the whole dose; this is not always easy, especially for older users and children. For this reason, prior art inhalators often do not obtain a full inhalation of the dose, but only a part thereof, with the remaining part being deposited in the inhalation conduit and the means for dosing, thus contaminating these areas and causing device blockage.

DE-A-101 06 788 discloses an inhalator having a combination of characteristics as set forth in the pre-characterizing portion of the appended claim 1. US-A-5 383 850, EP-A-0 424 790, DE-C-44 15 462, US-A-4 570 630, and AP-A-272 (NORTON HEALTHCARE LTD) disclose inhalators each having a combination of characteristics as set forth in the pre-characterizing portion of the appended claim 1, except for the feature of the cylindrical mobile portion 9.

The main aim of the present invention is to provide an inhalator for powder mixtures which obviates the drawbacks existing in the prior art.

An advantage of the invention is that the inhalator is extremely simple, reliable and economical.

A further advantage of the inhalator of the present invention is that it enables a full assumption of a dose of mixture with only a brief aspiration of modest intensity on the part of the user.

Further characteristics and advantages of the present invention will better emerge from the detailed description that follows of an inhalator for powder mixtures, illustrated purely by way of non-limiting example in the accompanying figures of the drawings, in which:

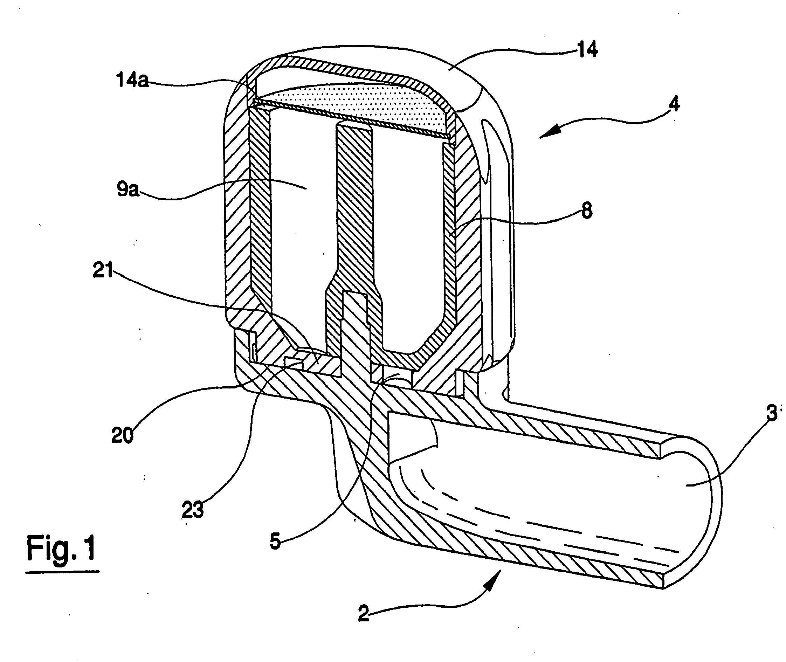

figure 1 shows a perspective view in section of the inhalator of the invention;

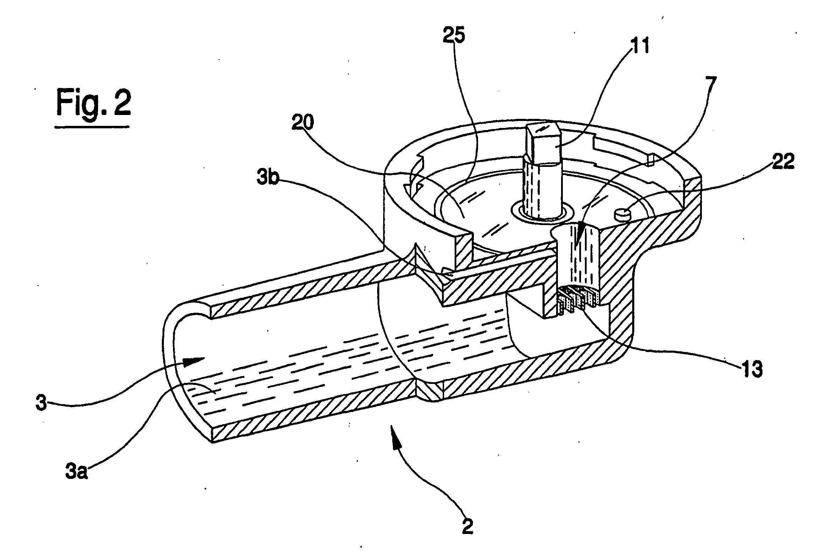

figure 2 is a perspective view in section of a component of the inhalator of figure 1;

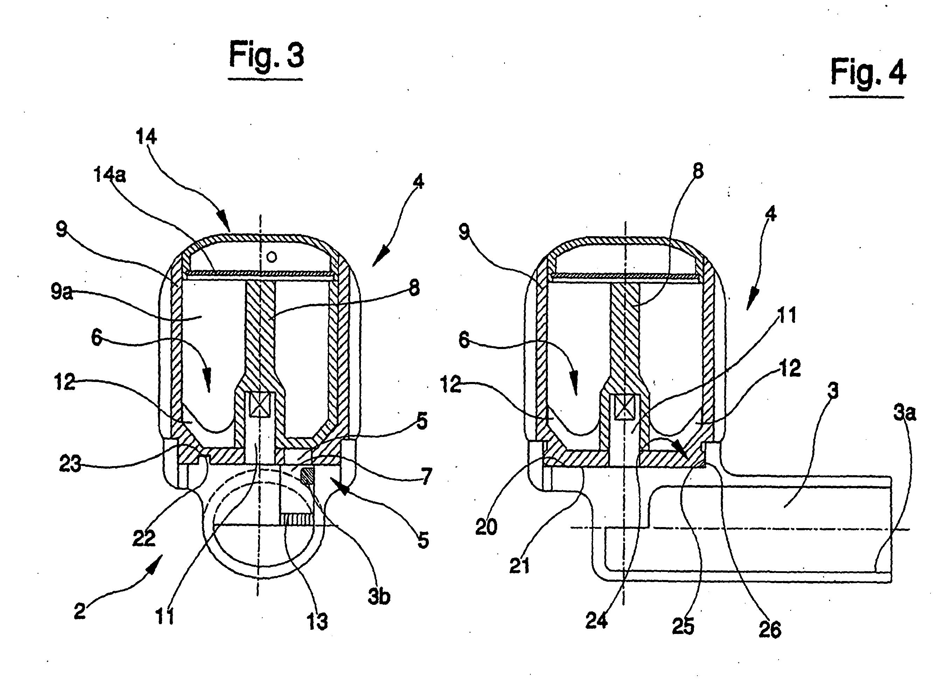

figure 3 is a front view of the inhalator of figure 1 in which some details are illustrated in section;
figure 4 is a view from the left of the inhalator of figure 3;
figure 5 is a first view from above of the inhalator of figure 3 in a first operative configuration;
figure 6 is a second view from above of the inhalator of figure 3 in a second operative configuration.

The figures of the drawings illustrate in their entirety an inhalator according to the present invention. It comprises a main body 2, provided with an inhalation channel 3 and a reservoir 4 for containing a powder mixture, arranged superiorly of the main body 2. A batching chamber 5, of predetermined size, is located between the inhalation channel 3 and the reservoir 4; the batching chamber 5 is mobile on command between a first position, in which it is not in communication with the inhalation channel 3, but is in communication with the reservoir 4 and receives the powder mixture, and a second position, in which it is not in communication with the reservoir 4 but is in communication with the inhalation channel 3 and releases the powder mixture by free fall into the inhalation chamber 3. The reservoir 4 is internally provided with means for mixing 6 predisposed to mix the powder preparation at each command given to the batching means 5 in order to avoid lumps forming in the mixture, which can obstruct and block, preventing the inhalator from working properly.

The inhalation channel 3 exhibits a first mouth 3a, predisposed for aspiration of air from inside the channel 3 and a second mouth 3b, predisposed for inlet of air into the channel 3. The inhalation channel 3 is superiorly further provided with an entrance hole 7 which places an internal part thereof in communication with the batching chamber 5. In the illustrated embodiment the inhalation chamber 3 substantially exhibits a C-shaped longitudinal development. The entrance hole 7 extends longitudinally in a perpendicular direction to two parallel tracts at ends of which the first and second mouths 3a and 3b are located.

The inlet hole 7 exhibits, at an end thereof facing towards the inhalation channel 3, a grid 13, the elements of which occupy the whole of the end and are of a size such as to prevent free passage of the powder but to allow a passage thereof following an aspiration from the inhalation channel 3. As will better emerge hereinafter, the function of the grid 13 is to restrain the powder mixture in arrival by free fall from the reservoir 4, so that it is completely invested by the flow of air induced by the user when aspirating.

The reservoir 4 comprises a mobile portion 9, which exhibits an internal cavity 9a of an overall cylindrical shape. The internal cavity 9a inferiorly exhibits a circular face 21 in which the batching chamber 5 is fashioned. The reservoir 4 also comprises a fixed portion 8 which is solid to the main body 2. The fixed portion 8 exhibits a cylindrical sector and is coaxial and laterally envelops the internal cavity 9a. The fixed portion 8 is arranged internally of the mobile portion 9 in order to limit the free volume of the internal cavity 9a and to lie above the entry hole 7. The fixed portion 8 is arranged above the circular face 21 and is in contact there-with, while laterally it is in contact with the lateral surface of the internal cavity 9a. The fixed portion 8 defines, with the walls of the internal cavity 9a, a cylindrical sector which is overall free and complementary to the shape of the fixed portion 8. The powder mixture contained internally of the reservoir 4 can occupy only the cylindrical sector. The reservoir 4 is superiorly closed by a cap 14 which is partially insertable in an upper tract of the mobile portion 9. The cap 14 exhibits an internal chamber which can contain a dehumidifying agent and is separate from the inside of the reservoir 4 thanks to a porous wall 14a predisposed to enable the humidity inside the reservoir 4 to be absorbed internally of the cap 14.

The main body 2 superiorly exhibits a circular surface 20 on which the tank 4 is arranged. A pivot 11 is located at a centre of the circular surface 20, at which centre the entrance hole 7 is afforded. The mobile portion 9 rotates about the pivot 11. The circular surface 20 is complementarily shaped with respect to the circular face 21 of the mobile portion 9 and is predisposed to slide on the face 21 in the rotation direction of the mobile portion 9. The circular face 21 is thus in contact on one side with the fixed portion 8 and on the other side with the circular surface 20. The batching chamber 5, which is afforded in the circular face 21 and is constituted for example by a circular through-hole, is thus superiorly delimited by the circular face 21 and inferiorly by the circular surface 20. The volume defined of the through-hole, the circular face 21 and the circular surface 20 is cylindrical and predisposed to contain a powder mixture dose.

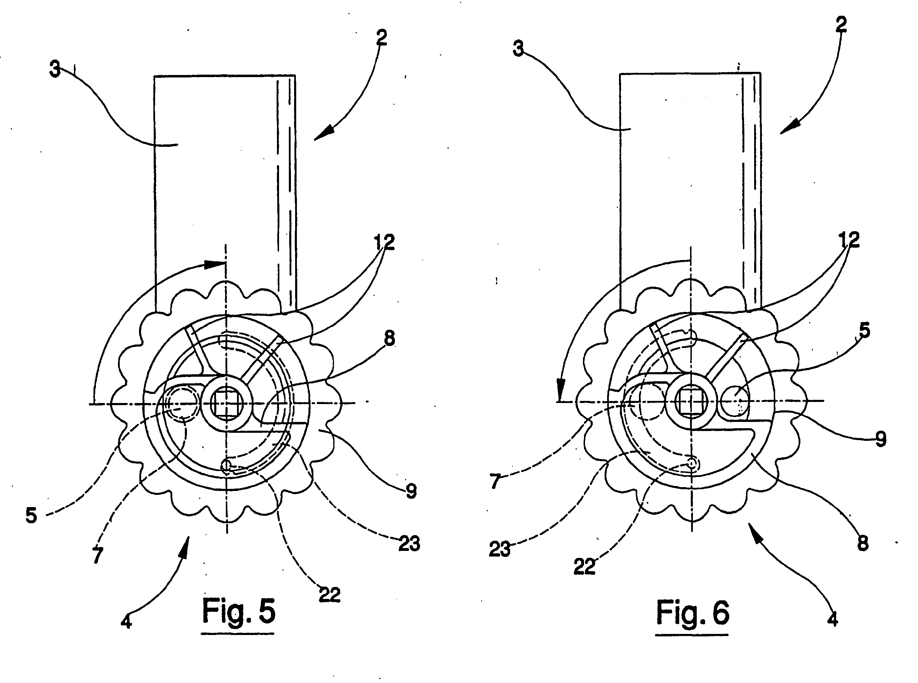

The mobile portion 9 is rotatable between two extreme positions about the pivot 11. At the first position the batching chamber 5 does not face the entrance hole 7 and is superiorly open towards the internal cavity 9a which is not occupied by the fixed portion 8. In the second position the batching chamber 5 faces the entrance hole 7 of the internal channel 3 and is superiorly closed by the fixed portion 8. The batching, or dosing, of the mixture is achieved by rotating the mobile portion 9. As can be seen in figure 6, when the mobile portion 9 is in the first position the batching chamber 5 is superiorly open and inferiorly closed by the circular surface 20. In this position the powder mixture enters the batching chamber 5. By rotating the mobile portion 9 from the first towards the second position, the batching chamber 5 fills completely with the powder mixture. The batching chamber 5, during the rotation of the mobile portion 9, describes an arc of circumference at least for a tract of which it is not superiorly closed by the fixed portion 8, but faces the internal cavity 9a. In the second position, the batching chamber 5 is superiorly closed by the fixed portion 8 and faces the entrance hole 7. The mixture which has filled the batching chamber 5 during the rotation of the mobile portion 9 can thus fall through the entrance hole 7 into the inhalation channel 3. Since in the second position the batching chamber 5 is superiorly closed by the fixed portion 8, only the quantity of mixture contained inside the chamber, which is the dose of mixture, is unloaded into the inhalation chamber 3. The dose which falls through the entrance hole 7 deposits on the grid 13 which is at an intermediate point of the length of the inhalation channel 3. The flow of air which moves from the second mouth 3b towards the first mouth 3a by effect of the user's aspiration, completely invests the grid 13 on which the dose of mixture is located, which batch is thence entrained towards the first mouth 3a. The conformation of the grid 13 increases the turbulence of the air flow, increasing the entraining action on the powder mixture dose.

In order to define the amount of the rotation of the mobile portion 9, the circular surface 20 of the main body 2 superiorly exhibits a pin 22 which engages in a channel 23 developing in an arc afforded inferiorly in the circular face 21. During rotations of the mobile portion 9, the pin 22 slides along the channel 23. The amount of the rotations is limited by the length of the channel 23, the ends of which function as endrun stops for the pin 22. When the pin 22 is in one of the extreme positions of the channel 23, the mobile portion is at, respectively, the first and second positions thereof.

The means for mixing 6 comprise at least one vane 12, solidly constrained to the fixed portion 8 and arranged superiorly of the batching chamber 5. In the illustrated embodiment there are two vanes 12 which project from the fixed portion 8 in a radial direction with respect to the internal cavity 9a and are inferiorly in contact with the circular face 21. The mixing of the powders is done at each rotation of the mobile portion 9; the rotation of the mobile portion 9 tends to cause the mixture to turn over inside the internal chamber 9a. As the vanes 12 are solidly constrained to the fixed portion 8 and do not rotate with the mobile portion 9, the powder mixture in proximity of the circular face 21 stops in contract with the vanes 12; in this way relative motion is created between the lower and upper layers of the mass of powder mixtures, thus avoiding the formation of lumps.

At least one seal 24 is positioned between the circular surface 20 of the main body 2 and the circular face 21 of the mobile portion 9. The seal 24 comprises a circular ribbing 25 which projects superiorly from the flat circular surface and is predisposed to insert in a groove 26 afforded inferiorly of the circular face 21. In the illustrated embodiment there are two concentric seals 24 having the function of preventing air from penetrating internally of the reservoir and contaminating the mixture, and preventing the mixture from penetrating between the circular face 21 and the circular surface 20, obstructing the rotation of the mobile portion 9.

The inhalator of the present invention offers some important advantages. Firstly, in accordance with the attached main claim 1, it is extremely simple and economical. It is composed of a limited number of parts, easily assembled, and does not require the predisposing of complex mechanisms. The only relative motion between the various parts is the rotation of the mobile portion 9 with respect to the fixed portion 8 and the main body 2. Thanks to this simplicity the inhalator is substantially free of blockages and damage resulting from use. In accordance with the attached dependent claims the present of the vanes 12 removes the eventuality of blockages and constantly guarantees release of a complete dose of powder mixture. The dose is positioned in an intermediate tract of the inhalation channel 3 in order to be completely invested by the flow of air crossing the device by effect of the user's aspiration. This characteristic of the invention ensures that the powder mixture dose is assumed by the user completely and with only a minimum force applied, a detail of particular importance when used by children or older people.

## Claims

1. An inhalator for powder mixtures, comprising a main body (2) provided with an inhalation channel (3) and a reservoir (4) for containing a powder mixture, said reservoir (4) being arranged superiorly of the main body (2) the inhalator further comprising a batching chamber (5) of a predefined volume, arranged between the inhalation channel (3) and the reservoir (4), and mobile on command between a first position, in which it is not in communication with the inhalation channel (3) and is in communication with the reservoir (4) for receiving the powder mixture, and a second position, in which it is not in communication with the reservoir (4) and is in communication with the inhalation channel (3) for releasing the powder mixture by free fall internally of the inhalation channel (3), the inhalator comprising a mobile portion (9) which exhibits an interval cavity (9a) having an overall cylindrical shape, which internal cavity (9a) inferiorly exhibits a circular face (21) in which the batching chamber (5) is afforded, **characterised in that**: the reservoir (4) comprises a fixed portion (8), solidly constrained to the main body (2), which exhibits a cylindrical sector shape and is coaxial and counter-shaped laterally to the interne cavity (9a); the fixed portion (8) is arranged internally of the mobile portion (9) in order to limit a free volume of the internal cavity (9a) and to overlie an entrance hole (7) provided superiorly on the inhalation channel (3) which places an internal part thereof in communication with the batching chamber (5).

2. The inhalator of claim 1, **characterised in that** the reservoir (4) is internally provided with means for mixing (6) predisposed to mix the powder mixture on each command to the batching chamber (5).

3. The inhalator of claim 1, **characterised in that** the inhalation channel (3) exhibits a first mouth (3a) predisposed for aspiration of air from inside the inhalation channel (3), and a second mouth (3b), predisposed for inlet of air internally of the inhalation channel (3).

4. The inhalator of claim 1, **characterised in that** the mobile portion (9) is rotatable between a first position, at which the batching chamber (5) faces the entrance hole (7) of the inhalation channel (3) and is superiorly closed by the fixed portion (8), and a second position, at which the batching chamber (5) does not face the entrance hole (7) and is superiorly open towards the internal cavity of the mobile portion (9) which is not occupied by the fixed portion (8).

5. The inhalator of claim 4, **characterised in that**: the reservoir (4) is internally provided with means for mixing (6) predisposed to mix the powder mixture at each command made on the batching chamber (5); the means for mixing (6) comprising at least one vane (12), solidly constrained to the fixed portion (8) and arranged at a higher level with respect to the batching chamber (5).

6. The inhalator of claim 3, **characterised in that** the entrance hole (7) exhibits, at an end thereof facing the inhalation channel (3), a grid (13), elements (13) of which occupy the end and are of a size sufficient to obstruct free passage of the powder mixture and also to allow passage of the powder mixture following aspiration in the inhalation channel (3).

7. The inhalator of claim 4, **characterised in that**: the main body (2) superiorly exhibits a circular surface (20) on which the reservoir (4) is arranged; a pivot (11) being arranged at a centre of the circular surface (20), about which pivot (11) the mobile portion (9) rotates; the entrance hole (7) opens on the flat circular surface (20).

8. The inhalator of claim 7, **characterised in that** the flat circular surface (20) is countershaped with respect to the circular face (21) and is predisposed to slide on the circular face (21) in a rotation direction of the mobile portion (9), the circular surface (20) of the main body (2) superiorly exhibiting a pin (22) predisposed to engage in a channel (23) developing in an arc and afforded inferiorly of the circular face (21).

9. The inhalator of claim 8, **characterised in that** between the circular surface (20) of the main body (2) and the circular face (21) of the mobile portion (9), at least one seal (24) is predisposed, which comprises a circular ribbing (25) which projects superiorly from the circular surface (20) and inserts in a groove. (26) afforded inferiorly in the circular face (21).

## Patentansprüche

1. Inhalator für pulverförmige Mixturen mit einem Hauptkörper (2), der mit einem Inhalationskanal (3) und einem Speicher (4) zur Vorhaltung einer pulverförmigen Mixtur ausgestattet ist, wobei der Speicher (4) oberhalb des Hauptkörpers (2) angeordnet ist und der Inhalator ferner eine Dosierkammer (5) eines bestimmten Volumens aufweist, die zwischen dem Inhalationskanal (3) und dem Speicher (4) angeordnet, und auf Anforderung beweglich zwischen einer ersten Position, in welcher sie nicht in Verbindung mit dem Inhalationskanal (3) und in Verbindung mit dem Speicher (4) zur Aufnahme der pulverförmigen Mixtur ist, und einer zweiten Position ist, in welcher sie nicht in Verbindung mit dem Speicher (4) und in Verbindung mit dem Inhalationskanal (3) zur Abgabe der pulverförmigen Mixtur durch freien Fall im Inneren des Inhalationskanals (3) ist, und wobei der Inhalator ein bewegliches Teil (9) aufweist, welches einen Innenraum (9a) mit einer im Wesentlichen zylindrischen Form aufweist, welcher Innenraum (9a) unten eine kreisförmige Fläche (21) aufweist, in welche die Dosierkammer (5) geschaffen ist, **dadurch gekennzeichnet, dass** der Speicher (4) ein ortsfestes Teil (8) aufweist, das mit dem Hauptkörper (2) fest verbunden ist, und welches die Form eines zylindrischen Sektors aufweist und koaxial und gegengleich zu dem Innenraum (9a) geformt ist; dass das ortsfeste Teil (8) innerhalb des beweglichen Teils (9) angeordnet ist, um ein freies Volumen des Innenraums (9a) zu begrenzen und eine Eintrittsöffnung (7) zu überdecken, die oberhalb der Inhalationskanal (3) angebracht ist, welche Eintrittsöffnung einen inneren Teil hiervon in Verbindung mit der Dosierkammer (5) bringt.

2. Inhalator nach Anspruch 1, **dadurch gekennzeichnet, dass** der Speicher (4) im Inneren mit Mitteln (6) zum Mixen ausgestattet ist, die dafür ausgelegt sind, die pulverförmige Mixtur bei jeder Anforderung der Dosierkammer (5) zu vermischen.

3. Inhalator nach Anspruch 1, **dadurch gekennzeichnet, dass** der Inhalationskanal (3) eine erste Öffnung (3a) aufweist, die für die Ansaugung von Luft aus dem Inneren des Inhalationskanals (3) vorgesehen ist, und eine zweite Öffnung (3b), die für die Zuleitung von Luft in das Innere des Inhalationskanals (3) vorgesehen ist.

4. Inhalator nach Anspruch 1, **dadurch gekennzeichnet, dass** das bewegliche Teil (9) zwischen einer ersten Position, in der die Dosierkammer (5) zur Eintrittsöffnung (7) des Inhalationskanals (3) hin ausgerichtet ist und darüber durch das ortsfeste Teil (8) verschlossen ist, und einer zweiten Position, in der die Dosierkammer (5) nicht zur Eintrittsöffnung (7) hin ausgerichtet ist und nach oben hin offen gegenüber dem Innenraum des beweglichen Teils (9) ist, welcher nicht von dem ortsfesten Teil (8) abgedeckt ist, rotierbar ist.

5. Inhalator nach Anspruch 4, **dadurch gekennzeichnet, dass** der Speicher (4) intern mit Mitteln (6) zum Mixen ausgestattet ist, um die pulverförmige Mixtur auf jede Anforderung an die Dosierkammer (5) zu mixen; dass die Mittel (6) zum Mixen zumindest eine Schaufel (12) aufweisen, die fest an dem ortsfesten Teil (8) angebracht ist und gegenüber der Dosierkammer (5) höher angeordnet ist.

6. Inhalator nach Anspruch 3, **dadurch gekennzeichnet, dass** die Eintrittsöffnung (7) an einem Ende, das in Richtung des Inhalationskanals (3) ausgerichtet ist, ein Gitter (13) aufweist, dessen Elemente (13) an dem Ende angeordnet sind und eine Größe haben, die ausreicht, um den freien Durchtritt der pulverförmigen Mixtur zu hindern und auch um den Durchtritt der pulverförmigen Mixtur infolge Ansaugung in den Inhalationskanal (3) zu ermöglichen.

7. Inhalator nach Anspruch 4, **dadurch gekennzeichnet, dass** der Hauptkörper (2) oben eine kreisförmige Oberfläche (20) aufweist, auf der der Speicher (4) angeordnet ist; dass eine Achse (11) in einem Zentrum der kreisförmigen Oberfläche (20) angeordnet ist, um welche das bewegliche Teil (9) rotiert; dass die Eingangsöffnung (7) auf der flachen kreisförmigen Oberfläche (20) mündet.

8. Inhalator nach Anspruch 7, **dadurch gekennzeichnet, dass** die flache kreisförmige Oberfläche (20) gegengleich zur kreisförmigen Fläche (21) ist und darauf ausgelegt ist, auf der kreisförmigen Fläche (21) in einer Drehrichtung des beweglichen Teils (9) zu gleiten, wobei die kreisförmige Oberfläche (20) des Hauptkörpers (2) oberhalb einen Stift (22) aufweist, der darauf ausgelegt ist, in eine Führung (23) einzugreifen, die sich in einem Bogen erstreckt und unten an der kreisförmigen Fläche (21) geschaffen ist.

9. Inhalator nach Anspruch 8, **dadurch gekennzeichnet, dass** zwischen der kreisförmigen Oberfläche (20) des Hauptkörpers (2) und der kreisförmigen Fläche (21) des beweglichen Teils (9) zumindest eine Dichtung (24) vorgesehen ist, welche eine kreisförmige Riffelung (25) aufweist, die oberhalb der kreisförmigen Oberfläche (20) vorsteht und in eine Fuge (26) eingebracht ist, die unterhalb in der kreisförmigen Fläche (21) geschaffen ist.

## Revendications

1. Inhalateur pour mélanges en poudre, comprenant un corps principal (2) muni d'un canal (3) d'inhalation et un réservoir (4) destiné à contenir un mélange en poudre, ledit réservoir (4) étant placé au-dessus du corps principal (2), l'inhalateur comprenant de plus une chambre de dosage (5) d'un volume prédéfini, placée entre le canal d'inhalation (3) et le réservoir (4), et mobile sur commande entre une première position, dans laquelle elle n'est pas en communication avec le canal d'inhalation (3) et est en communication avec le réservoir (4) afin de recevoir le mélange en poudre, et une deuxième position, dans laquelle elle n'est pas en communication avec le réservoir (4) et est en communication avec le canal d'inhalation (3) afin de libérer le mélange en poudre par une chute libre à l'intérieur du canal d'inhalation (3), l'inhalateur comprenant une portion mobile (9) qui présente une cavité interne (9a) ayant une forme d'ensemble cylindrique, la cavité interne (9a) présentant sur la partie inférieure une face circulaire (21) dans laquelle la chambre de dosage (5) est ménagée,
***caractérisé en ce que** :* le réservoir (4) comprend une portion fixe (8), solidement assujettie au corps principal (2), qui présente une forme en secteur cylindrique et est coaxiale et contre-profilée latéralement par rapport à la cavité interne (9a) ; la portion fixe (8) est située à l'intérieur de la portion mobile (9) de manière à limiter un volume libre de la cavité interne (9a) et à recouvrir un orifice d'entrée (7) ménagé sur le haut du canal d'inhalation (3) et qui place une partie interne de celui-ci en communication avec la chambre de dosage (5).

2. Inhalateur selon la revendication 1, ***caractérisé en ce que*** le réservoir (4) est muni intérieurement de moyens de mélange (6) prévus pour mélanger le mélange en poudre à chaque commande envoyée à la chambre de dosage (5).

3. Inhalateur selon la revendication 1, ***caractérisé en ce que*** le canal d'inhalation (3) présente une première ouverture (3a) prévue pour l'aspiration d'air en provenance de l'intérieur du canal d'inhalation (3) et une deuxième ouverture (3b) prévue pour l'entrée d'air à l'intérieur du canal d'inhalation (3).

4. Inhalateur selon la revendication 1, ***caractérisé en ce que*** la portion mobile (9) est rotative entre une première position, dans laquelle la chambre de dosage (5) fait face à l'orifice d'entrée (7) du canal d'inhalation (3) et est fermée sur le haut par la portion fixe (8), et une deuxième position, dans laquelle la chambre de dosage (5) ne fait pas face à l'orifice d'entrée (7) et s'ouvre sur le haut vers la cavité interne de la portion mobile (9) qui n'est pas occupée par la portion fixe (8).

5. Inhalateur selon la revendication 4, ***caractérisé en ce que**:* le réservoir (4) est muni intérieurement de moyens de mélange (6) prévus pour mélanger le mélange en poudre à chaque commande faite à la chambre de dosage (5) ; les moyens de mélange (6) comprenant au moins une ailette (12), solidement assujettie à la portion fixe (8) et placée à un niveau supérieur à celui de la chambre de dosage (5).

6. Inhalateur selon la revendication 3, ***caractérisé en ce que*** l'orifice d'entrée (7) présente, à une de ses extrémités faisant face au canal d'inhalation (3), une grille (13), dont des éléments (13) occupent l'extrémité et sont d'une taille suffisante pour obstruer le libre passage du mélange en poudre mais pour permettre aussi le passage du mélange en poudre à la suite d'une aspiration dans le canal d'inhalation (3).

7. Inhalateur selon la revendication 4, ***caractérisé en ce que** :* le corps principal (2) présente sur le haut une surface circulaire (20) sur laquelle est placé le réservoir (4), un pivot (11) étant placé au centre de la surface circulaire (20), pivot (11) autour duquel tourne la portion mobile (9) ; l'orifice d'entrée (7) s'ouvre sur la surface circulaire plate (20).

8. Inhalateur selon la revendication 7, ***caractérisé en ce que*** la surface circulaire plate (20) est contre-profilée par rapport à la face circulaire (21) et est prévue pour glisser sur la face circulaire (21) dans une direction de rotation de la portion mobile (9), la surface circulaire (20) du corps principal (2) présentant sur le dessus un ergot (22) prévu pour s'engager dans une gorge (23) se développant en arc et ménagée sur le dessous de la face circulaire (21).

9. Inhalateur selon la revendication 8, ***Caractérisé en ce que**,* entre la surface circulaire (20) du corps principal (2) et la face circulaire (21) de la portion mobile (9), au moins un joint d'étanchéité (24) est prévu, qui comprend un nervurage circulaire (25) qui fait saillie sur le dessus de la surface circulaire (20) et s'insère dans une rainure (26) ménagée sur le dessous dans la face circulaire (21).
